# EUROPÄISCHE PATENTSCHRIFT

(11) **EP 0 838 702 B1**
(45) Veröffentlichungstag und Bekanntmachung des Hinweises auf die Patenterteilung: **25.10.2006**
(21) Anmeldenummer: 97810663.1
(22) Anmeldetag: 12.09.1997
(51) Int. Cl.: G02B 6/38

(54) **Stecker für eine optische Steckverbindung**
Connector for an optical connection
Connecteur pour une connexion optique

(30) Priorität: 28.10.1996 CH 264696
(43) Veröffentlichungstag der Anmeldung: 29.04.1998
(73) Patentinhaber: Diamond SA, 6616 Losone (CH)
(72) Erfinder: De Marchi, Silverio, 6612 Ascona (CH); Marazzi, Silvio, 6600 Locarno (CH)
(74) Vertreter: Wenger, René

(56) Entgegenhaltungen:
- EP-A- 0 613 030
- FR-A- 2 439 409
- GB-A- 2 112 173
- PATENT ABSTRACTS OF JAPAN vol. 003, no. 015 (E-089), 9.Februar 1979 & JP 53 141644 A (FUJITSU LTD), 9.Dezember 1978,
- "Fiber optic connector with built-in shutter" RESEARCH DISCLOSURE, März 1987, SALEM ,MA.,USA, Seite 124 XP000645314

## Beschreibung

Die Erfindung betrifft einen Stecker für eine optische Steckverbindung gemäss dem Oberbegriff von Anspruch 1. Die Aufgabe der die Stirnseite bedeckenden Schutzklappe besteht dabei primär darin, die Endfläche des Lichtwellenleiters vor mechanischer Einwirkung zu schützen und um das Austreten von Licht im ausgesteckten Zustand zu verhindern.

Schutzklappen an optischen Steckern sind bereits seit einiger Zeit bekannt und gebräuchlich. Bei einigen bekannten Steckern ist die Schutzklappe gelenkig mit dem Steckergehäuse verbunden. So beispielsweise beim Stecker gemäss EP-A-613 030, bei dem die Schutzklappe um seitliche Lagerbolzen schwenkbar ist, welche in Lagerbohrungen eingerastet sind. Beim Einstecken des Steckers in ein Buchsenteil laufen ebenfalls seitlich angeordnete Führungsnocken an der Schutzklappe auf einer Führungskulisse am Buchsenteil auf und bewirken so die Öffnungsbewegung der Schutzklappe. Eine derartige Schutzvorrichtung eignet sich nicht für alle Steckverbindungen, insbesondere nicht für solche, bei denen ohne separates Buchsenteil unmittelbar gleichartige Stecker miteinander gepaart werden sollen.

Durch "Research Disclosure" (1987) März, Nr. 275, New York, ein gattungsmässig vergleichbarer Stecker bekannt geworden, bei dem eine einstückig mit dem Steckergehäuse ausgebildete Blende beim Einstecken in ein Buchsenteil durch eine korrespondierende Steuerfläche seitlich weggedrückt wird. Beim Ausstecken des Steckers federt die Blende infolge ihrer Materialelastizität wieder zurück und deckt die Stirnseite ab. Ein Nachteil dieser Bauweise besteht darin, dass die Blende oder Schutzklappe aus dem gleichen Material hergestellt werden muss, wie das Steckergehäuse. Es wäre jedoch wünschenswert, dass das Steckergehäuse zur Fassung der Lichtwellenleiter aus einem sehr harten Material besteht. Ein weiterer Nachteil der bekannten Konstruktion besteht darin, dass infolge der einstückigen Ausbildung die Schutzklappe die Stirnseite des Steckers lediglich im Abstand überdeckt (Blendenwirkung), jedoch zur Vermeidung von Schmutzansammlung nicht gegen die Stirnseite gepresst werden kann.

Die bekannten Stecker mit Schutzklappen betreffen zumeist nur Stecker für einen einzigen Lichtwellenleiter. In letzter Zeit werden jedoch vermehrt optische Flachbandkabel mit einer Mehrzahl von Lichtwellenleitern eingesetzt, welche spezielle Stecker erfordern. Bei diesen sogenannten MT-Steckern sind an der Stirnseite mehrere Endflächen von Lichtwellenleitern in einer Reihe angeordnet. Ein typischer MT-Stecker ist beispielsweise in der EP-A-226 274 beschrieben. Brauchbare Schutzklappen für MT-Stecker, welche beim Steckvorgang automatisch öffnen, sind bisher nicht bekannt.

Es ist daher eine Aufgabe der Erfindung, einen Stecker der eingangs genannten Art zu schaffen, der konstruktiv einfach aufgebaut ist und bei dem die Schutzklappe die Stirnseite zuverlässig vor Verschmutzung schützt. Die Schutzklappenkonstruktion soll sich insbesondere für den Einsatz an MT-Steckern für Flachbandkabel eignen. Diese Aufgabe wird erfindungsgemäss mit einem Stecker gelöst, der die Merkmale im Anspruch 1 aufweist.

Die vom Steckergehäuse lösbare Anordnung des als Biegefeder ausgebildeten Hebelarms hat gegenüber der bekannten, einstückigen Ausbildung mehrere Vorteile. Einerseits kann das gleiche Steckergehäuse auch ohne Schutzklappe verwendet werden, was in zahlreichen Anwendungsfällen erwünscht ist. Anderseits erlaubt es die separate Anordnung aber auch, den Hebelarm mit der Schutzklappe aus einem anderen Material herzustellen, als das Steckergehäuse, wobei optimale Federeigenschaften angestrebt werden können. Insbesondere lässt sich die Schutzklappe so ausbilden und am Steckergehäuse fixieren, dass die Stirnseite nicht nur im Sinne einer Blende überdeckt wird, sondern dass sich die Schutzklappe federnd gegen die Stirnseite presst und damit ein Eindringen von Staub und Schmutz verhindert wird. Zu diesem Zweck ist die Schutzklappe in der Schliessstellung unter Federvorspannung gegen die Stirnseite gepresst.

Weitere Vorteile lassen sich erreichen, wenn die Stirnseite und die ihr zugewandte Innenfläche der Schutzklappe quer zu einer Tangentenfläche verlaufen, welche den von der Schutzklappe beschriebenen Kreisbogen im Bereich der Endfläche des Lichtwellenleiters berührt. Die Stirnseite und die ihr zugewandte Innenfläche der Schutzklappe kann dabei um 5 bis 10°, vorzugsweise um etwa 8° zu einer Ebene geneigt sein, welche im rechten Winkel zur optischen Achse des Lichtwellenleiters verläuft. Bei dieser Anordnung wird nicht nur eine Anpressung der Innenfläche der Schutzklappe gegen die Stirnseite des Steckers ermöglicht, sondern die Schutzklappe bewirkt beim Zurückfedern in die Schliessstellung ein Wegschieben von Verschmutzungen auf der Stirnseite, etwa vergleichbar mit einem Scheibenwischer. Es ist dabei durchaus denkbar, dass die Stirnseite und die Innenfläche geringfügig unterschiedliche Winkel aufweisen, um diesen Effekt noch zu verstärken.

Eine besonders einfache Befestigung des Hebelarms am Steckergehäuse wird dadurch erreicht, dass dieser mittels Rastmitteln am Steckergehäuse eingerastet ist. Dadurch wird die Montage erheblich erleichtert und es sind keinerlei zusätzliche Befestigungsmittel wie Schrauben oder dergleichen erforderlich.

Vorzugsweise besteht der Hebelarm und die Schutzklappe einstückig aus einem Kunststoffmaterial, ebenso wie das Steckergehäuse, wobei das Steckergehäuse aus einem Kunststoffmaterial ausgebildet ist, das eine geringere Elastizität aufweist als dasjenige des Hebelarms und der Schutzklappe. Während beim Hebelarm die Federeigenschaften im Vordergrund stehen, soll das Gehäuse mechanisch resistent sein und die Lichtwellenleiter präzise fassen. Letztere Eigenschaften erfordern ein relativ hartes Kunststoffmaterial.

Bei einem MT-Stecker, bei dem an der Stirnseite die Endflächen mehrerer Lichtwellenleiter in Reihe angeordnet sind, ist es besonders vorteilhaft, wenn die Schutzklappe leistenförmig ausgebildet ist und damit sämtliche Lichtwellenleiter gleichzeitig überdeckt.

Insbesondere bei einer leistenförmigen Ausbildung der Schutzklappe ist es vorteilhaft, wenn der Hebelarm etwa U-förmig ausgebildet ist, wobei die Enden der U-Schenkel im Steckergehäuse eingerastet sind und zum Einrasten und Lösen federnd gegeneinander pressbar sind.

Dabei können an den Enden der U-Schenkel aussenseitige Sperrklinken oder Wulste angeordnet sein, welche in Ausnehmungen am Steckergehäuse eingerastet sind. Der Hebelarm mit der Schutzklappe bildet dabei eine Art Gabel, wobei die Gabelschenkel zum Einsetzen in das Steckergehäuse zusammengedrückt werden können. Auch hier spielt die richtige Wahl der Materialelastizität eine entscheidende Rolle.

Die Ausnehmungen können parallele Hohlkehlen sein, welche sich gegen die Stirnseite hin öffnen, wobei zur Sicherung zwischen den U-Schenkeln ein Sperrstück am Steckergehäuse angeordnet ist, das von den Enden der U-Schenkel innenseitig hinterfasst wird. Diese einseitig geöffneten Ausnehmungen lassen sich spritzgusstechnisch einfach herstellen. Das Sperrstück verhindert ein Herausgleiten der U-Schenkel und dient gleichzeitig der lagemässigen Orientierung der Schutzklappe.

Die Öffnungsbewegung, d.h. ein federndes Zurückschwenken der Schutzklappe, lässt sich besonders einfach realisieren, wenn die Schutzklappe auf der von der Stirnseite abgewandten Seite eine Auflauffläche aufweist, welche derart geneigt ist, dass beim Auflaufen auf eine Rampe an einem Steckergegenstück die Schutzklappe weggeschwenkt wird. Die Schutzklappe weist somit gleichzeitig eine Steuerfläche auf, welche die Schwenkbewegung beim Steckvorgang steuert. Im Bereich der Stirnseite kann dabei zudem eine Rampe angeordnet sein, an welcher die Schutzklappe eines gleichartigen Steckergegenstücks weggeschwenkt wird.

Schliesslich ist es auch besonders vorteilhaft, wenn die Rampe in eine Anlagefläche übergeht, auf der die Schutzklappe eines gleichartigen Steckergegenstücks nach dem Erreichen der Endstellung anliegt. Die auf den Gegenstecker einwirkende Federkraft kann somit bezüglich ihrer Kraftrichtung derart umgelenkt werden, dass sie die Anpresskraft der gegeneinander gepressten Stecker nicht reduziert. Bei entsprechender Ausbildung der Anlagefläche ist es sogar denkbar, dass diese Anpresskraft noch vergrössert wird.

Das Steckergehäuse kann etwa quaderförmig ausgebildet sein und der schwenkbare Hebelarm kann innerhalb des Quaders in einer Ausnehmung gehalten sein. Auf diese Weise werden die Aussenabmessungen des Steckers durch die Anordnung des Hebelarms nicht vergrössert. Bei dieser versenkten Bauweise wird auch weitgehend verhindert, dass die Schutzklappe im ausgesteckten Zustand unbeabsichtigt aufgeschwenkt wird.

Die einzelnen Stecker sind vorzugsweise so ausgebildet, dass mit einem Steckerpaar das bezogen auf die Steckerachse bzw. auf die optische Achse des oder der Lichtwellenleiter um 180° zueinander verdreht ist, eine Steckverbindung herstellbar ist, bei welcher die Schutzklappen bei einer Relativbewegung der Stecker aufeinander zu durch jeweils gleiche Bauteile des Gegensteckers simultan wegschwenkbar sind.

Ein Ausführungsbeispiel der Erfindung ist in den Zeichnungen dargestellt und wird nachstehend genauer beschrieben. Es zeigen:
- Figur 1: eine perspektivische Darstellung von zwei um 180° zueinander verdrehten Steckern vor dem Einsetzen der Schutzklappen,
- Figur 2: die beiden Stecker gemäss Figur 1 mit eingesetzten Schutzklappen,
- Figur 3: die beiden Stecker gemäss Figur 2 kurz vor dem Einsteckvorgang,
- Figuren 4 bis 8: Querschnitte durch die Steckverbindung in verschiedenen Phasen des Öffnungsvorgangs der Schutzklappen, und
- Figur 9: ein Querschnitt durch das Steckergehäuse mit den geometrischen Bedingungen an der Steckerstirnseite.

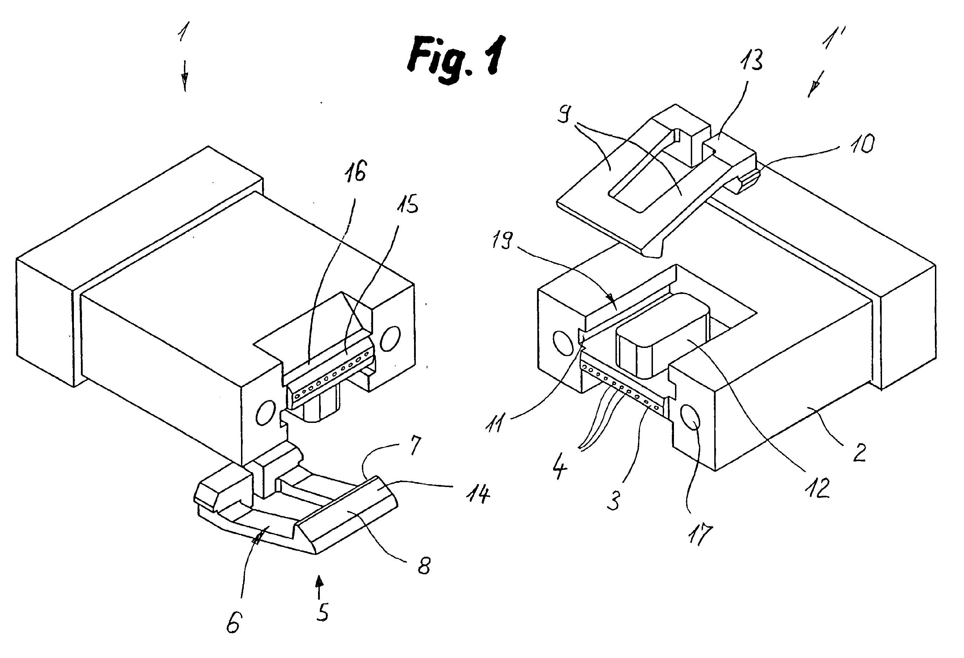

Wie aus Figur 1 ersichtlich ist, besteht ein Stecker 1 bzw. 1' im wesentlichen aus einem Steckergehäuse 2, an dessen Stirnseite 3 die Endflächen mehrerer Lichtwellenleiter 4 in einer Reihe angeordnet und gehalten sind. Es handelt sich somit um einen MT-Stecker für optische Flachbandkabel, wobei die Erfindung jedoch nicht auf diesen Steckertyp beschränkt ist. Die Art und Weise, wie das Flachbandkabel im Steckergehäuse befestigt wird, ist dem Fachmann bekannt und wird hier nicht näher beschrieben.

Das Steckergehäuse ist im wesentlichen quaderförmig ausgebildet und auf einer Seite mit einer Ausnehmung 19 versehen, die gegen die Stirnseite 3 hin geöffnet ist. In den Seitenwänden der Ausnehmung sind Hohlkehlen 11 angeordnet, die sich ebenfalls gegen die Stirnseite hin öffnen. Im Zentrum der Ausnehmung ist ein Sperrstück 12 angeordnet, dessen Oberfläche etwa mit der Oberfläche des Steckergehäuses fluchtet.

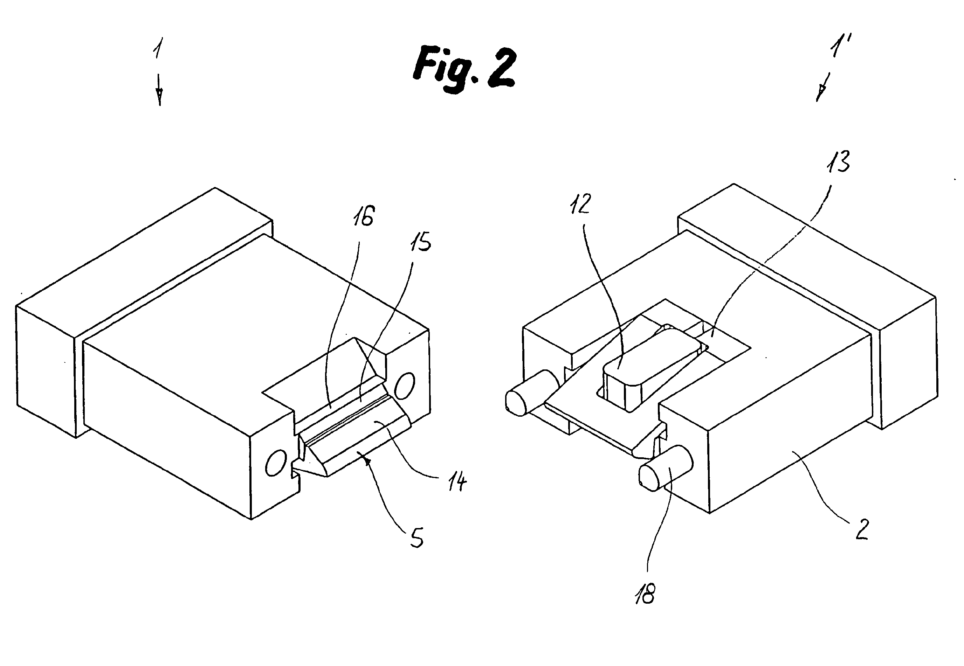

Etwa auf der gleichen Ebene wie die Lichtwellenleiter 4 sind seitliche Bohrungen 17 angeordnet, welche der Aufnahme von Zentrierstiften 18 (Figur 2) dienen.

Unmittelbar im Bereich der Stirnseite 3 ist an jedem Stecker eine Rampe 15 angeordnet, welche in eine Anlagefläche 16 übergeht. Die Anlagefläche 16 verläuft dabei etwa parallel zur Ebene der Lichtwellenleiter 4.

Jedem Stecker ist eine Schutzklappe 5 zugeordnet, die im wesentlichen als Leiste 8 ausgebildet ist und die eine Innenfläche 7 aufweist, welche gegen die Stirnseite 3 pressbar ist. Die Schutzklappe ist einstückig mit zwei U-Schenkeln 9 ausgebildet, welche insgesamt einen Hebelarm 6 bilden, um welchen die Schutzklappe federnd schwenkbar ist. Der Hebelarm ist dabei als Biegefeder ausgebildet, wobei auch nur ein Abschnitt des Hebelarms beispielsweise im Bereich der Einspannstelle als Biegegelenk ausgebildet sein kann.

An der Aussenseite der Leiste 8 ist eine Auflauffläche 14 angeordnet, welche dazu dient, beim Steckvorgang die Schwenkbewegung der Schutzklappe einzuleiten. An den Enden der U-Schenkel 9 sind seitlich Sperrklinken 10 angeordnet, welche in die seitlichen Hohlkehlen 11 einrastbar sind. Innenseitig ist jeder U-Schenkel mit einer Sperrnase 13 versehen, welche das Sperrstück 12 am Steckergehäuse hintergreift.

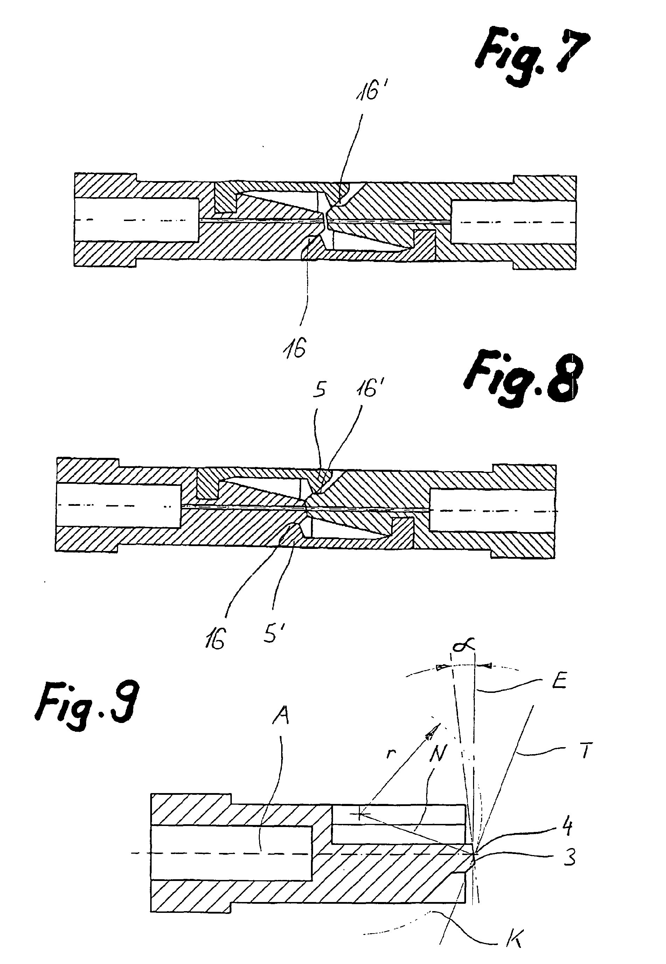

Zum Einsetzen der Schutzklappen werden die U-Schenkel 9 etwas zusammengedrückt, so dass die Sperrklinken 10 in den Hohlkehlen 11 einrasten. Aus Figur 2 ist ersichtlich, dass die U-Schenkel 9 in eingerasteter Position unter einem Winkel schräg zur Oberfläche des Steckergehäuses verlaufen. In dieser Position wird jeweils die Innenfläche 7 unter Federvorspannung gegen die Stirnseite 3 gepresst. Einzelheiten der geometrischen Anordnung der Stirnseite 3 sind aus Figur 9 ersichtlich. Die Stirnseite 3 verläuft demnach quer zu einer Tangente T, welche den Kreisbogen K der Schutzklappe im Bereich der Endflächen der Lichtwellenleiter 4 tangiert. Die Normale N zu dieser Tangente verläuft somit zwischen einem virtuellen Drehpunkt des Hebelarms 6 und den Endflächen der Lichtwellenleiter 4. Gleichzeitig verläuft die Stirnseite 3 aber auch geneigt zu einer Ebene E, welche im rechten Winkel zur optischen Achse A der Lichtwellenleiter 4 verläuft. Der Neigungswinkel α beträgt 5° bis 10°, vorzugsweise etwa 8°.

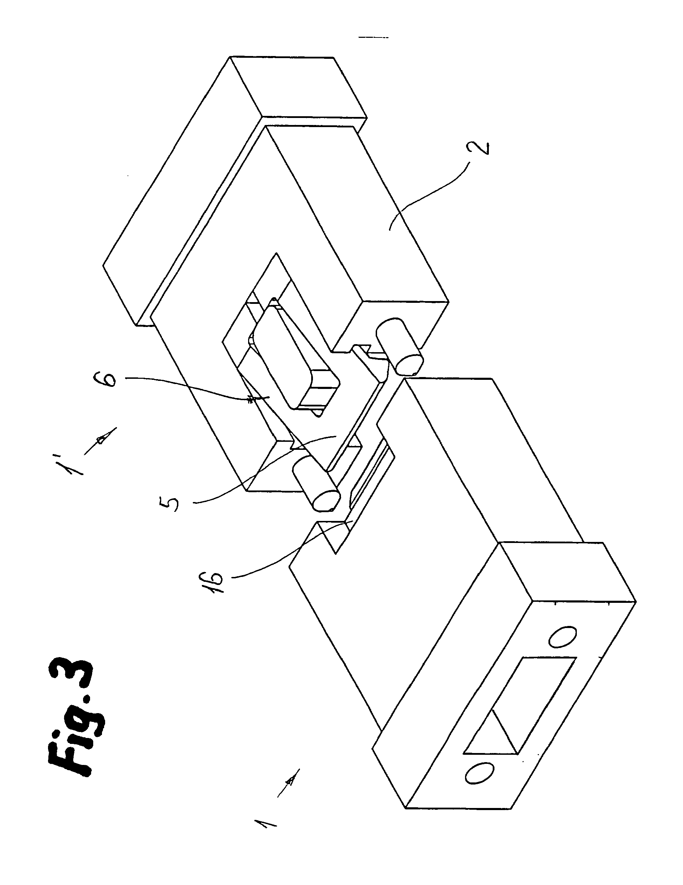

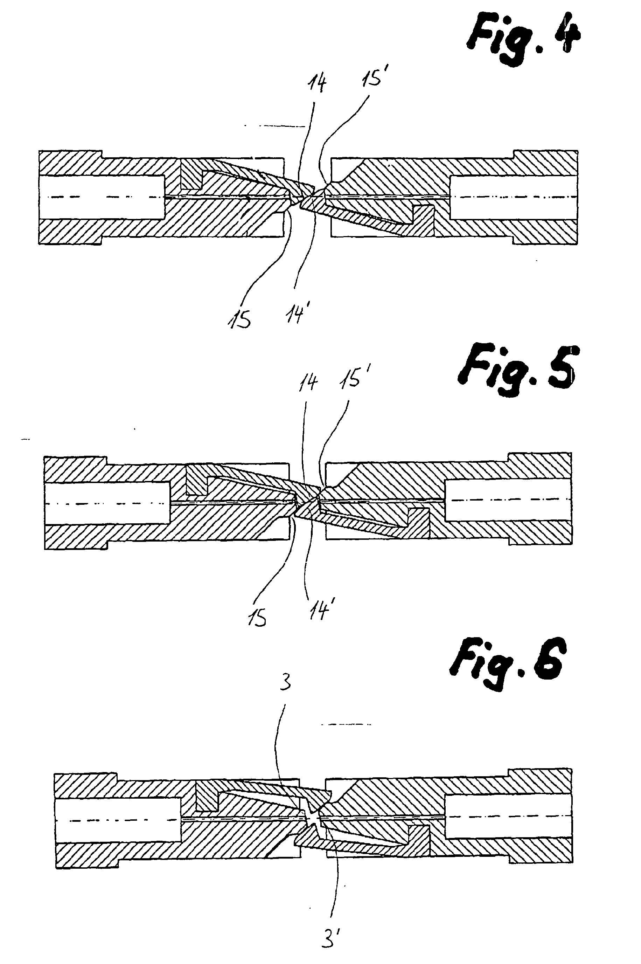

Der Öffnungsvorgang der Schutzklappen beim zusammenstecken gleichartiger Stecker wird nun anhand der Figuren 4 bis 8 beschrieben. Die Ausgangsposition ist dabei zunächst in Figur 3 dargestellt, in welcher die Zentrierstifte 18 noch nicht in die Bohrung 17 des gegenüberliegenden Steckers eingedrungen sind. Nach dem eigentlichen Zentriervorgang berühren sich zunächst die Auflaufflächen 14, 14' der Schutzklappen, wie in Figur 4 dargestellt ist.

Bei einer weiteren Relativbewegung der beiden Stecker aufeinander zu beginnen sich die Schutzklappen gegenseitig aus der Ruhestellung wegzuschwenken, wobei die Auflaufflächen 14, 14' die Rampen 15, 15' am jeweiligen Gegenstück erreichen. Diese Position ist in Figur 5 dargestellt.

Die weitere Schwenkbewegung erfolgt nun ausschliesslich an den Rampen, wie aus Figur 6 ersichtlich ist, wobei nun auch die Blendenwirkung der Schutzklappen aufgehoben wird.

Kurz bevor die Stirnseiten 3, 3' einander berühren erreichen die Schutzklappen das Ende der Rampen (Figur 7) und gleiten auf korrespondierende Auflageflächen 16, 16', wo sie auch nach dem gegeneinanderpressen der Stirnseiten verbleiben (Figur 8). Aus der Darstellung ist ersichtlich, dass die Federkraft an den Schutzklappen 5 und 5' jetzt annähernd im rechten Winkel auf die Auflaufflächen 16, 16' wirkt und dabei infolge der Schräglage der Stirnflächen 3, 3' letztere noch gegeneinander presst. Die beiden Stecker werden durch hier nicht dargestellte Mittel im eingesteckten Zustand gegeneinander gepresst, wobei diese Anpresskraft jedoch durch die Federkraft an den Schutzklappen nicht reduziert oder aufgehoben wird.

Selbstverständlich wären alternative Ausbildungen der Schutzklappe denkbar, ohne das erfindungsgemäss Prinzip zu verlassen. So könnten beispielsweise für einzelne Lichtwellenleiter oder Gruppen von Lichtwellenleitern separate Schutzklappen am Steckergehäuse angeordnet sein. Es wäre auch denkbar, dass die Schutzklappen mit anderen Mitteln als durch entsprechende Führungskulissen am Steckergegenstück weggeschwenkt werden. Denkbar wäre eine separate Öffnungsvorrichtung, welche jeweils vor dem Einsteckvorgang manuell betätigt wird.

## Patentansprüche

1. Stecker (1) für eine optische Steckverbindung, mit einem Steckergehäuse (2), an dessen Stirnseite (3) die Endfläche wenigstens eines Lichtwellenleiters (4) gehalten ist, sowie mit einer die Stirnseite in einer Schliessstellung bedeckenden Schutzklappe (5), welche derart federnd am Ende eines als Biegefeder ausgebildeten Hebelarms (6) am Steckergehäuse (2) gehalten ist, dass sie gegen die Federkraft der Biegefeder in eine die Stirnseite (3) freilegende Öffnungsstellung schwenkbar ist, **dadurch gekennzeichnet, dass** der Hebelarm (6) lösbar mit dem Steckergehäuse (2) verbunden ist und dass die Schutzklappe (5) in der Schliessstellung unter Federvorspannung der Biegefeder gegen die Stirnseite (3) gepresst ist.

2. Stecker nach Anspruch 1, **dadurch gekennzeichnet, dass** die Stirnseite (3) und die ihr zugewandte Innenfläche (7) der Schutzklappe (5) quer zu einer Tangentenfläche (T) verlaufen, welche den von der Schutzklappe (5) beschriebenen Kreisbogen (K) im Bereich der Endfläche des Lichtwellenleiters (4) berührt.

3. Stecker nach Anspruch 1 oder 2, **dadurch gekennzeichnet, dass** die Stirnseite (3) und die ihr zugewandte Innenfläche (7) der Schutzklappe (5) um 5° bis 10°, vorzugsweise um etwa 8° zu einer Ebene (E) geneigt ist, welche im rechten Winkel zur optischen Achse (A) des Lichtwellenleiters (4) verläuft.

4. Stecker nach einem der Ansprüche 1 bis 3, **dadurch gekennzeichnet, dass** der Hebelarm (6) mittels Rastmitteln (10) am Steckergehäuse (2) eingerastet ist.

5. Stecker nach einem der Ansprüche 1 bis 4, **dadurch gekennzeichnet, dass** der Hebelarm (6) und die Schutzklappe (5) einstückig aus einem Kunststoffmaterial ausgebildet sind und dass das Steckergehäuse ebenfalls aus einem Kunststoffmaterial ausgebildet ist, das eine geringere Elastizität aufweist, als dasjenige des Hebelarms und der Schutzklappe.

6. Stecker nach einem der Ansprüche 1 bis 5, **dadurch gekennzeichnet, dass** an der Stirnseite (3) die Endflächen mehrerer Lichtwellenleiter (4) in einer Reihe angeordnet sind und dass die Schutzklappe (5) leistenförmig ausgebildet ist.

7. Stecker nach einem der Ansprüche 1 bis 6, **dadurch gekennzeichnet, dass** der Hebelarm (6) etwa U-förmig ausgebildet ist, wobei die Enden der U-Schenkel im Steckergehäuse (2) eingerastet sind und zum Einrasten und Lösen federnd gegeneinander pressbar sind.

8. Stecker nach Anspruch 7, **dadurch gekennzeichnet, dass** an den Enden der U-Schenkel (9) aussenseitige Sperrklinken (10) oder Wulste angeordnet sind, welche in Ausnehmungen (11) am Steckergehäuse (2) einrastbar sind.

9. Stecker nach Anspruch 8, **dadurch gekennzeichnet, dass** die Ausnehmungen parallele Hohlkehlen (11) sind, welche sich gegen die Stirnseite (3) hin öffnen und dass zur Sicherung zwischen den U-Schenkeln (9) ein Sperrstück (12) am Steckergehäuse angeordnet ist, das von den Enden der U-Schenkel innenseitig hinterfasst wird.

10. Stecker nach einem der Ansprüche 1 bis 9, **dadurch gekennzeichnet, dass** die Schutzklappe (5) auf der von der Stirnseite (3) abgewandten Seite eine Auflauffläche (14) aufweist, welche derart geneigt ist, dass beim Auflaufen auf eine Rampe (15) an einem Steckergegenstück die Schutzklappe (5) weggeschwenkt wird.

11. Stecker nach Anspruch 10, **dadurch gekennzeichnet, dass** im Bereich der Stirnseite (3) eine Rampe (15) zum Wegschwenken der Schutzklappe eines gleichartigen Steckergegenstücks angeordnet ist.

12. Stecker nach Anspruch 11, **dadurch gekennzeichnet, dass** die Rampe (15) in eine Anlagefläche (16) übergeht, auf der die Schutzklappe eines gleichartigen Steckergegenstücks nach dem Erreichen einer Endstellung anliegt.

13. Steckverbindung mit zwei gleichartigen Steckern (1, 1') nach einem der Ansprüche 10 bis 12, wobei die Stecker bezogen auf die Steckerachse um 180° zueinander verdreht sind und wobei die Schutzklappen bei einer Relativbewegung der Stecker aufeinander zu durch jeweils gleiche Bauteile des Gegensteckers simultan wegschwenkbar sind.

14. Stecker nach einem der Ansprüche 1 bis 12, **dadurch gekennzeichnet, dass** das Steckergehäuse (2) etwa quaderförmig ausgebildet ist und dass der schwenkbare Hebelarm (6) innerhalb des Quaders in einer Ausnehmung (19) gehalten ist.

## Claims

1. Plug (1) for an optical plug-in connection, comprising a plug housing (2), at the front end (3) of which the end face of at least one optical waveguide (4) is held, and with a protective flap (5) covering the front end in a closed position, which is held elastically at the plug housing (2) at the end of a lever arm (6) constructed as flexural spring in such a manner that it can be swivelled against the spring force of the flexural spring into an opening position exposing the front end (3), **characterized in that** the lever arm (6) is detachably connected to the plug housing (2) and that the protective flap (5) is pressed against the front end (3) under spring pretension of the flexural spring in the closing position.

2. Plug according to Claim 1, **characterized in that** the front end (3) and the inner face (7) of the protective flap (5) facing it extend transversely to a tangent surface (T) which touches the circular arc (K) described by the protective flap (5) in the area of the end face of the optical waveguide (4).

3. Plug according to Claim 1 or 2, **characterized in that** the front end (3) and the inner surface (7) of the protective flap (5) facing it is inclined by 5° to 10°, preferably by about 8° to a plane (E) which extends at a right angle to the optical axis (A) of the optical waveguide (4).

4. Plug according to one of Claims 1 to 3, **characterized in that** the lever arm (6) is locked in at the plug housing (2) by means of locking means (10).

5. Plug according to one of Claims 1 to 4, **characterized in that** the lever arm (6) and the protective flap (5) are constructed in one piece of a plastic material and **in that** the plug housing is also constructed of a plastic material which has a lesser elasticity than that of the lever arm and of the protective flap.

6. Plug according to one of Claims 1 to 5, **characterized in that** the end faces of a number of optical waveguides (4) are arranged in a row at the front end (3) and **in that** the protective flap (5) is constructed in strip form.

7. Plug according to one of Claims 1 to 6, **characterized in that** the lever arm (6) is constructed to be approximately U-shaped, the ends of the U legs being locked in the plug housing (2) and being able to be pressed elastically against one another for locking in and detaching.

8. Plug according to Claim 7, **characterized in that** at the ends of the U legs (9), outside latches (10) or beads are arranged which can be locked into recesses (11) on the plug housing (2).

9. Plug according to Claim 8, **characterized in that** the recesses are parallel grooves (11) which open towards the front end (3) and **in that**, for the purpose of securing, a blocking piece (12), behind which the ends of the U legs engage on the inside, is arranged between the U legs (9) at the plug housing.

10. Plug according to one of Claims 1 to 9, **characterized in that** the protective flap (5) has on the side facing away from the front end (3) a run-up surface (14) which is inclined in such a manner that when running up onto a ramp (15) on a plug counterpart, the protective flap (5) is swivelled away.

11. Plug according to Claim 10, **characterized in that** in the area of the front end (3), a ramp (15) is arranged for swivelling away the protective flap of a similar flap counterpart.

12. Plug according to Claim 11, **characterized in that** the ramp (15) changes into bearing surface (16) on which the protective flap of a similar plug counterpart rests after reaching an end position.

13. Plug-in connection with two similar plugs (1, 1') according to one of Claims 10 to 12, wherein the plugs are rotated with respect to one another by 180° with reference to the plug axis and wherein the protective flaps, in the case of a relative movement of the plugs towards one another can be simultaneously swivelled away by in each case identical components of the matching plug.

14. Plug according to one of Claims 1 to 12, **characterized in that** the plug housing (2) is constructed to be approximately cube-shaped and that the swivellable lever arm (6) is held in a recess (19) within the cube.

## Revendications

1. Fiche (1) pour une connexion par fiche optique, comprenant un boîtier de fiche (2), sur le côté avant (3) duquel la face d'extrémité d'au moins un guide optique (4) est maintenue, et un volet de protection (5) recouvrant le côté avant dans une position de fermeture, qui est maintenu de façon élastique sur l'extrémité d'un bras de levier (6) conçu comme ressort de flexion sur le boîtier de fiche (2) de telle sorte qu'il peut basculer contre la force de ressort du ressort de flexion dans une position d'ouverture libérant le côté avant (3), **caractérisée en ce que** le bras de levier (6) est relié de façon amovible au boîtier de fiche (2) et **en ce que** le volet de protection (5) est pressé dans la position de fermeture sous pré-tension du ressort de flexion contre le côté avant (3).

2. Fiche selon la revendication 1, **caractérisée en ce que** le côté avant (3) et la face intérieure (7), tournée vers ce côté, du volet de protection (5) s'étend transversalement à une surface de tangente (T), qui touche l'arc de cercle (K) décrit par le volet de protection (5) dans la zone de la surface d'extrémité du guide optique (4).

3. Fiche selon la revendication 1 ou 2, **caractérisée en ce que** le côté avant (3) et la face intérieure (7), tournée versce côté, du volet de protection (5) est incliné de 5° à 10°, de préférence d'environ 8° par rapport à un plan (E) qui s'étend à angle droit par rapport à l'axe optique (A) du guide optique (4).

4. Fiche selon l'une quelconque des revendications 1 à 3, **caractérisée en ce que** le bras de levier (6) est encliqueté à l'aide de moyens d'encliquetage (10) sur le boîtier de fiche (2).

5. Fiche selon l'une quelconque des revendications 1 à 4, **caractérisée en ce que** le bras de levier (6) et le volet de protection (5) sont conçus d'une seule pièce dans un matériau plastique et **en ce que** le boîtier de fiche est conçu également dans un matériau plastique, qui présente une élasticité plus faible que celle du bras de levier et du volet de protection.

6. Fiche selon l'une quelconque des revendications 1 à 5, **caractérisée en ce que** sur le côté avant (3) sont disposées les surfaces d'extrémité de plusieurs guides optiques (4) dans une rangée et **en ce que** le volet de protection (5) est conçu en forme de baguette.

7. Fiche selon l'une quelconque des revendications 1 à 6, **caractérisée en ce que** le bras de levier (6) est conçu à peu près en forme de U, les extrémités des branches du U étant encliquetées dans le boîtier de fiche (2) et pouvant être pressées les unes contre les autres de façon élastique pour l'encliquetage et le détachement.

8. Fiche selon la revendication 7, **caractérisée en ce que** des cliquets de blocage (10) ou des renflements, qui peuvent être encliquetés dans des évidements (11) sur le boîtier de fiche (2), sont disposés sur les extrémités des branches du U (9).

9. Fiche selon la revendication 8, **caractérisée en ce que** les évidements sont des gorges creuses (11) parallèles, qui s'ouvrent en direction du côté avant (3) et **en ce que**, pour le blocage entre les branches du U (9), une pièce de blocage (12) est disposée sur le boîtier de fiche qui est saisie par l'arrière côté intérieur par les extrémités des branches du U.

10. Fiche selon l'une quelconque des revendications 1 à 9, **caractérisée en ce que** le volet de protection (5) présente sur le côté opposé au côté avant (3) une surface d'arrivée (14), qui est inclinée de telle sorte que, lors de l'arrivée sur une rampe (15), le volet de protection (5) est basculé sur une pièce inverse de fiche.

11. Fiche selon la revendication 10, **caractérisée en ce qu'**une rampe (15) est disposée dans la zone du côté avant (3) pour le basculement du volet de protection d'une pièce inverse de fiche similaire.

12. Fiche selon la revendication 11, **caractérisée en ce que** la rampe (15) fait place à une surface d'appui (16), sur laquelle le volet de protection d'une pièce inverse de fiche similaire s'appuie après avoir atteint une position extrême.

13. Connexion par fiche avec deux fiches (1, 1') similaires, selon l'une quelconque des revendications 10 à 12, les fiches étant tournées l'une par rapport à l'autre de 180° par rapport à l'axe de la fiche et les volets de protection pouvant être basculés simultanément lors d'un déplacement relatif des fiches l'une vers l'autre par des composants respectivement identiques de la contre-fiche.

14. Fiche selon l'une quelconque des revendications 1 à 12, **caractérisée en ce que** le boîtier de fiche (2) est réalisé à peu près en forme de parallélépipède et **en ce que** le bras de levier (6) pivotant est maintenu à l'intérieur du parallélépipède dans un évidement (19).
